# EUROPÄISCHE PATENTSCHRIFT

(11) **EP 0 604 761 B1**
(45) Veröffentlichungstag und Bekanntmachung des Hinweises auf die Patenterteilung: **28.10.1998**
(21) Anmeldenummer: 93118817.1
(22) Anmeldetag: 23.11.1993
(51) Int. Cl.: A61B 17/00

(54) **Vorrichtung zum Verschliessen einer Öffnung in einem Gefäss**
Device for closing an opening in a vessel
Dispositif pour obstruer une ouverture dans un vaisseau

(30) Priorität: 23.11.1992 CS 3455/92
(43) Veröffentlichungstag der Anmeldung: 06.07.1994
(73) Patentinhaber: GOLLIEZ BIO-TEC Ltd., CH-1796 Courgevaux (CH)
(72) Erfinder: Krajicek, Milan Mudr CSc, Praha 4 (CS)
(74) Vertreter: Beetz & Partner Patentanwälte

(56) Entgegenhaltungen:
- EP-A- 0 415 087
- EP-A- 0 535 506
- WO-A-93/08740
- FR-A- 2 625 897
- US-A- 5 047 024
- US-A- 5 061 274

## Beschreibung

Die Erfindung betrifft eine Vorrichtung zum Verschließen einer Öffnung in einem Gefäß, z. B. einer Arterie, die durch Einführen eines Katheters verursacht wurde.

Bei der Diagnostik und Therapie von Gefäß-, Herz- und anderer Erkrankungen werden häufig Katheter eingesetzt, die transkutan in das Luminum des Gefäßes eingeführt werden und verschiedene Außendurchmesser - gemessen in "French" - haben können, wobei ein French etwa 0,3 mm ist. Am häufigsten werden Katheter mit einem Durchmesser von sechs bis acht French angewandt. In neuerer Zeit werden sog. "Sheets" verwendet, die ein etwa 10 bis 15 cm langes Kunststoffrohr besitzen, dessen lichte Weite dem Außendurchmesser des Katheters entspricht. Die Wand des Katheters ist sehr dünn, etwa 0,1 mm. Dabei wird nach der Punktion der Arterie bzw. des Gefäßes mit einer dünnen Nadel ein dünner Metalleiter mit dieser Nadel eingeführt und die Nadel wird entfernt. Mittels dieses Metalleiters wird dann in das Gefäß ein Katheter mit oder ohne Sheet eingeführt.

Nach der Beendigung der Untersuchung oder der Therapie wird der Katheter abgenommen, wobei in der Wand des Gefäßes eine Öffnung von entsprechenden Abmessungen verbleibt. Da in etwa 90 % aller Fälle eine Arterie punktiert wird, treten häufig durch den Druck in den Arterien markante Nachblutungen auf. Eine spontane Verschließung der Öffnung durch Blutgerinnsel wird erreicht, indem auf die Arterie in diesem Bereich etwa 20 Minuten lang ein manueller Druck ausgeübt wird. Ferner soll der Patient während der folgenden 24 Stunden liegen, wobei nach Punktionen z. B. im Leistenbereich ein gepolstertes Gewicht von etwa 1 bis 2 kg, z. B. ein Sandsack, auf die punktierte Stelle gelegt wird. Dieses Verfahren hat u. a. den Nachteil, daß der Patient unter ärztlicher Überwachung liegen muß, was eine kostspielige stationäre Behandlung im Krankenhaus bedeutet. Ferner können Komplikationen eintreten, wenn eine Nachblutung nicht vollständig und dauerhaft unterbunden werden kann, was einen chirurgischen Eingriff mit einer unmittelbaren Sutur der Gefäßwand erforderlich macht. In weniger häufigen Fällen ist die Kompression zu intensiv, wobei das gebildete Gerinnsel nicht nur die Öffnung in der Gefäßwand sperrt, sondern auch das Gefäß selbst teilweise oder vollständig verschließt. Auch in diesem Fall ist ein chirurgischer Eingriff unerläßlich. In beiden Fällen verlängert sich die stationäre Behandlung mit den Nachteilen einer Blockade von Klinikbetten und eines erheblichen Kostenanstiegs.

Gemäß einem im Versuchsstadium befindlichen Vorschlag zur Überwindung dieser Probleme wird vor der Punktion der Arterie der Abstand der Haut zum Gefäß gemessen. Nach dem Abnehmen des Katheters wird nach den Angaben des Meßgerätes ein besonderer Applikator zur Wand des Gefäßes geführt und mit diesem Applicator wird ggf. amorphes Kollagen einige Minuten lang eingepreßt, um die Gefäßöffnung zu verschließen.

Bei einem anderen aus der US 5 061 274 bekannten Verfahren werden ein Anker aus Polymer, ein Kollagen-Stöpsel und ein resorbierbarer Faden verwendet. Nach Entfernen des Katheters wird der Anker mit dem Faden in die Gefäßwand eingeführt und mit diesem Faden wird der Kollagen-Stöpsel über die Öffnung fixiert.

Der Hauptnachteil beider Verfahren besteht darin, daß sie erst nach der Entfernung des Katheters oder Sheets angewandt werden können und Blutungen während ihrer Durchführung nicht unterbunden Herden können, so daß die Gefahr einer Hämatombildung besteht. Nachteilig ist ferner, daß die Stelle des so behandelten Gefäßes innerhalb eines Monats nicht für weitere Punktionen herangezogen werden kann. Das zweite Verfahren ist kompliziert und kann nur von chirurgischem Fachpersonal durchgeführt werden. Schließlich sind beide Verfahren zeit- und kostenaufwendig, so daß insbesondere in Ländern mit niedrigem Niveau des Sanitätswesens die Kosten für die Hilfsmittel den Aufwand für einen eintägigen Klinikaufenthalt übersteigen können.

Aus der FR-A-2 625 897 ist es bekannt, einen Stöpsel auf einer in ein Blutgefäß eingeführten schlauch- bzw. rohrförmigen medizinischen Sonde anzuordnen und ihn mit Hilfe von Zangen auf dieser Sonde so weit zu verschieben, bis der kegelförmige Stöpsel mit seinem schmaleren vorderen Endabschnitt die Gefäßöffnung durchragt und damit den zwischen der Außenwandung der Sonde und der Gefäßöffnung gebildeten Spalt abdichtet. Zur verbesserten Positionierung des kegelförmigen Stopfens in der Gefäßöffnung sind an seiner Außenwandung zahn- bzw. rippenförmige Erhebungen ausgebildet.

Ferner wird in der - nachveröffentlichten - WO 93/08740 eine Vorrichtung zum Verschließen einer durch Einführen z.B. eines Katheters verursachten Gefäßöffnung vorgeschlagen, bei welcher ein oder mehrere kurze Rohrstücke aus einem amorphen Kollagen-Material innerhalb eines rohrförmigen Gehäuses angeordnet und zusammen mit diesem auf einer rohr- oder schlauchförmigen medizinischen Sonde, z.B. einem Katheter, positioniert werden. In dem Gehäuse sind ein rohrförmiger Stöpßel sowie eine Antriebseinheit zum axialen Verschieben dieses Stößels vorgesehen. Dieses Gehäuse wird auf dem Katheter so weit vorgeschoben, bis die innere Öffnung des rohrförmigen schmaleren Gehäuseabschnittes sich unmittelbar vor der Gefäßöffnung befindet. Nach Herausziehen des Katheters aus der Gefäßöffnung wird der rohrförmige Stöpßel im Gehäuse durch Betätigen des Antriebsmechanismus vorgeschoben, wodurch der rohrförmige Kollagen-Stöpsel aus der vorderen Gehäuseöffnung herausgedrückt und an der Gefäßwandung im Bereich der Gefäßöffnung positioniert wird. Das Kollagen quillt unter Einwirkung der Körperflüssigkeit auf, so daß der Innenraum des Stöpsels ausgefüllt und damit die Gefäßöffnung verschlossen wird. Der Inhalt dieser Anmeldung gehört zum Stand der Technik wie er im Artikel 54 (3) EPÜ definiert ist.

Aufgabe der Erfindung ist es, eine Vorrichtung zu schaffen, mit der eine z. B. durch Einführen eines Katheters verursachte Gefäßwand zuverlässig und auf einfache kostengünstige Weise verschlossen werden kann, um Nachblutungen zu vermeiden.

Diese Aufgabe wird durch die im Patentanspruch 1 angegebenen Merkmale gelöst.

Der Erfindung liegt die Erkenntnis zugrunde, daß der im Gefäß liegende Katheter als Führungsmittel für ein resorbierbares Verschlußelement dienen kann, das auf einem freien Katheterabschnitt außerhalb der Haut des Patienten plaziert wird und durch geeignete Hilfsmittel auf dem Katheter im Gewebekanal des Katheters bis zur Gefäßöffnung verschoben werden kann. Um die Gefäßöffnung nach Herausziehen des Katheters sicher zu verschließen, sollte das Material des Verschlußelements volumenvergrößernde Eigenschaften haben, damit der nach dem Herausziehen des Katheters entstandene Hohlraum ausgefüllt und auch eine sichere Abdichtung zwischen der Außenwand des Verschlußglieds und dem Rand der Gefäßöffnung erzielt wird.

Verwirklicht ist dieses Konzept in einem bevorzugten Ausführungsbeispiel mittels eines resorbierbaren Kollagen-Stöpsels als Verschlußglied, der einen durchgehenden V-förmigen Längsschlitz aufweist, der am äußeren Ende des Stöpsels weiter als an seinem inneren Endabschnitt ist. Die innere lichte Weite des Kollagen-Stöpsels, d. h. sein Innendurchmesser, entspricht dem Außendurchmesser des jeweiKatheters. Zum manuellen Verschieben des Kollagen-Stöpsels dient ein im folgenden als Applikator bezeichnetes hohlzylindrisches Bauteil, dessen Form und Wandstärke denjenigen des Kollagen-Stöpsels entsprechen und der ebenfalls einen in Axialrichtung V-förmigen durchgehenden Längsschlitz aufweisen kann, dessen Aufweitung am äußeren Ende größer als am inneren patientennahen Endabschnitt ist.

Vorteilhafte Weiterbildungen und Ausgestaltungen der erfindungsgemäßen Vorrichtung sind Gegenstand der Unteransprüche, wobei in einem weiteren Anspruch ein bevorzugtes Verfahren zur Herstellung eines Verschlußgliedes angegeben ist.

Durch die erfindungsgemäße Vorrichtung ergeben sich eine Reihe von gravierenden Vorteilen für den Patienten und auch für die ärztliche Behandlung. Der frühzeitige und sichere Verschluß der Gefäßöffnung innerhalb weniger Minuten verringert wesentlich die Gefahr von durch Nachblutungen verursachten Komplikationen, z. B. Infektionen, die bisher regelmäßig chirurgische Eingriffe und eine längere stationäre Behandlung des Patienten erforderlich machten. Ferner erübrigt sich die langzeitliche Auflage von beschwerlichen Druckpolstern. Neben den gravierenden Erleichterungen für den Patienten lassen sich erhebliche Kosteneinsparungen mit der aus nur wenigen einfachen Einzelteilen bestehenden Vorrichtung erzielen, weil nur ein geringer Zeitaufwand des behandelnden Arztes zum Einführen und Fixieren der Vorrichtung aufzubringen ist und die stationäre Behandlung entfallen oder zumindest erheblich abgekürzt werden kann.

Im folgenden werden bevorzugte Ausführungsbeispiele der Erfindung anhand der Zeichnung im einzelnen erläutert. Es zeigen:
- Fig. 1: einen in ein Gefäß eingeführten Katheter mit einer Vorrichtung gemäß der Erfindung;
- Fig. 2: schematisch einen zweischichtigen resorbierbaren Kollagen-Stöpsel;
- Fig. 3: einen starren Applikator aus inertem Werkstoff;
- Fig. 4: schematisch einen zur Anwendung mit einem Sheet geeigneten zweiteiligen Applikator;
- Fig. 5: teilgeschnitten einen profilierten Ring und
- Fig. 6: schematisch einen Teil eines Ansatzes zum Ansetzen des Applikators nach Fig. 4.

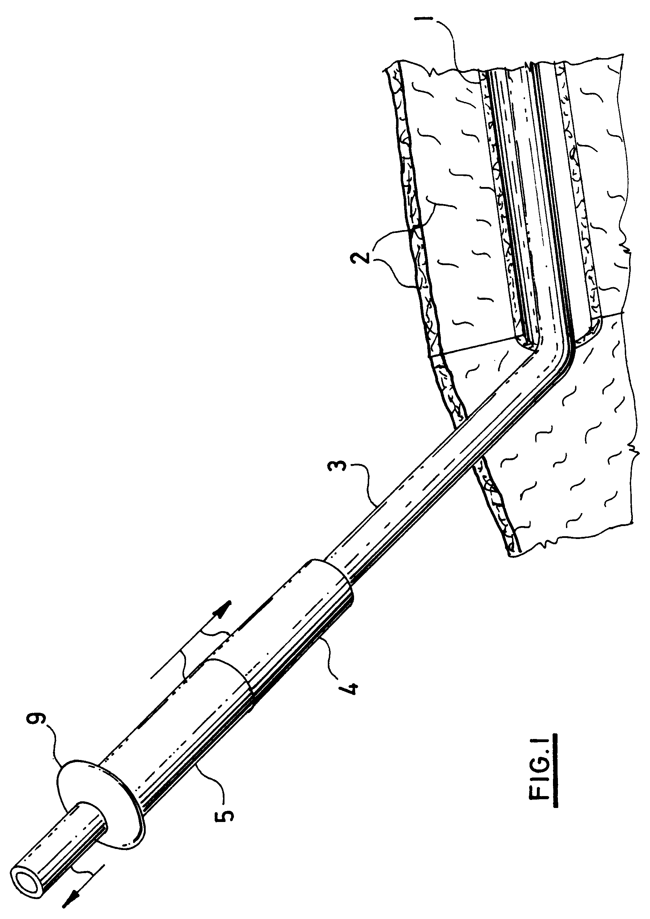

Die Vorrichtung zum Verschließen einer Gefäßöffnung weist einen rohrförmigen resorbierbaren Kollagen-Stöpsel auf, der aus zwei Schichten von komprimierten Kollagen-Schäumen besteht. Eine innere Schicht 6 ist aus einem relativ weicheren und daher schneller aufquellenden Schaum gefertigt und bildet etwa 80 % der gesamten Wandstärke des Stöpsels 4. Eine äußere Schicht 7 besteht aus einem stärker ausgehärteten und daher langsamer aufquellenden Schaum und bildet etwa 20 % der gesamten Wandstärke. Beide Schichten 6, 7 sind fest gegeneinandergedrückt. Der rohrförmige zweischichtige Kollagen-Stöpsel 4 hat einen Innendurchmesser, der dem Außendurchmesser eines flexiblen Katheters 3 (Fig. 1) oder eines starren kurzen Katheters 16, der Sheet genannt wird, entspricht.

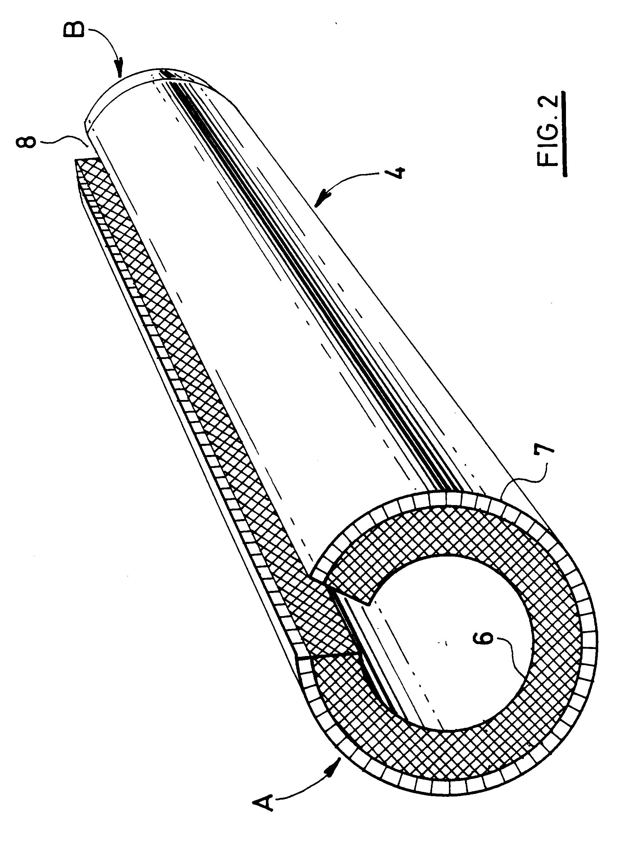

Wie aus Fig. 2 ersichtlich, hat der Kollagen-Stöpsel 4 einen seitlichen Schlitz 8, der sich über die gesamte Länge erstreckt und die Form eines schmalen V hat, wobei die Aufweitung dieses V an dem äußeren Ende A des Stöpsels mit Bezug auf das Gefäß 1 liegt und sich höchstens über ein Viertel des Außenumfangs erstreckt. Das andere Ende B des Stöpsels 4 ist mäßig geschärft, um sein Eindringen in den von der Haut 2 zum Gefäß 1 führenden Kanal des Katheters 3 zu erleichtern. Vor dem Einführen ist der Kollagen-Stöpsel 4 insbesondere durch seine Außenschicht 7 ausreichend starr. Die Aufquellung des Kollagen-Schaumes beginnt erst durch Einwirkung der Körperflüssigkeit, d. h. des Blutes und der Gewebeflüssigkeit, wobei zuerst die innere Schicht 6 in ca. 30 s und später innerhalb von etwa 2 min auch die äußere Schicht 7 aufquellen. Dieser zweiphasige Quellvorgang gewährleistet ein zuverlässiges und einfaches Einpressen des Kollagen-Stöpsels 4 bis unmittelbar zur Gefäßwand 1, weil die äußere Schicht 7 eine ausreichende Zeitspanne ihre Formsteifigkeit beibehält.

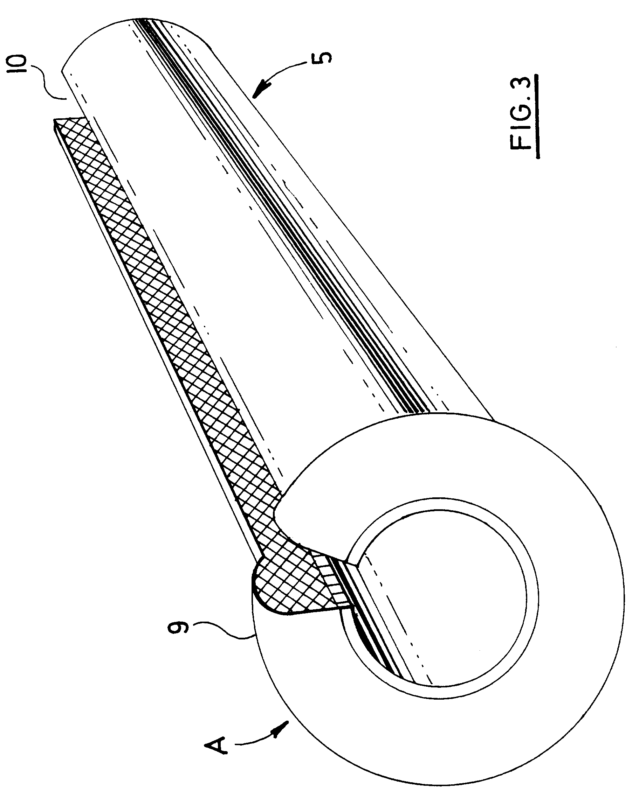

Ein zweiter Teil der Vorrichtung wird von einem in Fig. 3 dargestellten Applikator 5 aus starrem biologisch inerten und sterilisierbaren Werkstoff, z. B. aus Kunststoff, rostfreiem Stahl u. dgl., gebildet, der im wesentlichen eine gleiche Form und Wandstärke wie der Kollagen-Stöpsel 4 hat.

Auch der rohrförmige Applikator 5 weist einen V-förmigen Längsschlitz 10 auf, dessen größere Aufweitung sich am äußeren Ende A mit Bezug auf das Gefäß 1 befindet und sich nicht über ein Viertel des Umfangs dieses äußeren Endes A erstreckt. An diesem äußeren Ende A ist ein äußerer Ringbund bzw. Wulst 9 ausgebildet, der zur Abstützung der Finger, z. B. eines Daumens, beim Eindrücken des Kollagen-Stöpsels 4 entlang des Katheters 3 bis zur Gefäßwand dient.

Für jeden Außendurchmesser der Katheter 3 oder 16 gibt es Kollagen-Stöpsel 4 und Applikatoren 5 mit jeweils angepaßtem Innendurchmesser. Beide Teile 4 und 5 werden in zweifacher sicherer Verpackung und durch Bestrahlung sterilisert ausgeliefert.

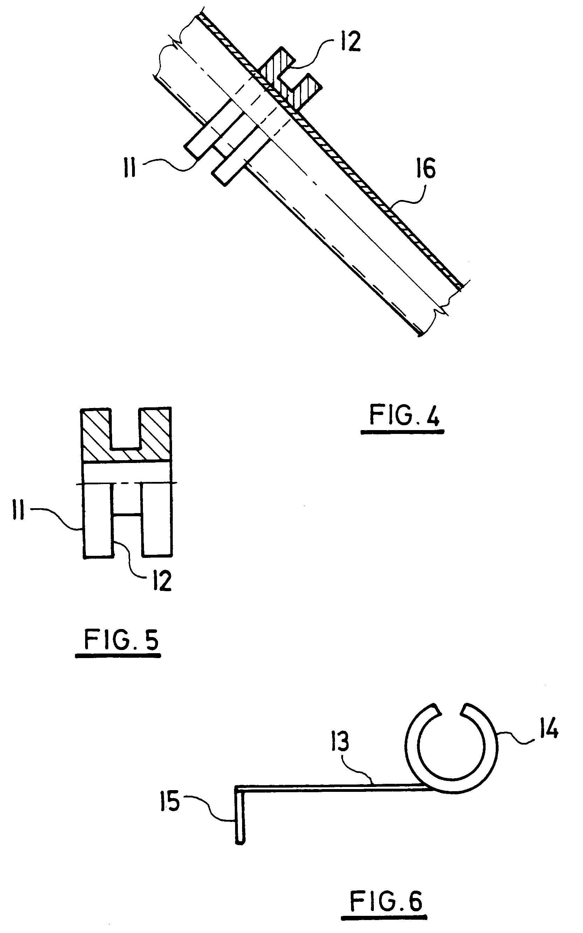

Die in Fig. 4 bis 6 dargestellte Variante wird mit einem äußeren kurzen Katheter 16, d. h. mit einem Sheet, eingesetzt, der wesentlich kürzer ist und nicht unmittelbar an eine Pumpe oder eine andere entsprechende Vorrichtung angeschlossen wird. Daher kann vor seiner Anwendung am Patienten ein Profilring 11 als Applikator auf den Kurzkatheter 16 von seinem Ende her aufgeschoben werden. Da die Abmessungen des Applikators bei der Handhabung Schwierigkeiten bereiten könnten, besteht dieser aus zwei Teilen, nämlich aus dem auf den Katheter 16 aufsetzbaren Profilring 11, der an seinem Außenumfang eine ausreichend tiefe Ringnut 12 aufweist. Vor der Anwendung wird in diese Ringnut 12 ein längsgeschlitzter Ring 14 seitlich eingedrückt, der am einen Ende eines abstehenden Ansatzes 13 befestigt ist. Dadurch kann über den Profilring 11 auf den Katheter 16 mittels eines am freien Ende des Ansatzes 13 vorgesehenen Glieds 15 vom Daumen ein ausreichender Druck erzeugt werden und nach der Applikation kann der Profilring 11 aus dem Gewebe herausgezogen werden.

Die Handhabung der erfindungsgemäßen Vorrichtung zum Verschließen eines Gefäßes bzw. einer Öffnung in einer Gefäßwand ist folgende.

Vor dem Entfernen eines durch eine Punktion durch die Haut 2a und das Gewebe 2b in ein Blutgefäß 1, insbesondere in eine Arterie, eingeführten Katheters 3 wird auf dessen frei herausragenden Abschnitt ein Kollagen-Stöpsel 4 durch Aufweiten seines V-förmigen Längsschlitzes 8 - beginnend mit dem Ende A - seitlich aufgedrückt. Auf gleiche Weise erfolgt die Anordnung eines relativ formsteifen Applikators 5 nach Fig. 1 und 3 durch seitliches Aufdrücken auf den angrenzenden äußeren Abschnitt des Katheters 3. Anschließend wird durch Drücken des Daumens gegen den endseitigen Ringwulst 9 der Applikator 5 zusammen mit dem Kollagen-Stöpsel 4 auf dem Katheter 3 so weit vorgeschoben, bis der Kollagen-Stöpsel 4 mit seinem vorderen Endteil in das Gefäß 1 eingedrungen ist. Danach wird der Katheter 3 aus dem Gefäß 1 herausgezogen, wobei der Daumendruck auf den Applikator 5 und damit auch auf den Kollagen-Stöpsel 4 aufrechterhalten wird, um den Kollagen-Stöpsel 4 in seiner Lage festzuhalten und sein Herausziehen aus dem Gefäß 1 zu verhindern. Bereits während und insbesondere nach dem Herausziehen des Katheters 3 wird der Kollagen-Stöpsel 4 mit Blut bzw. Gewebeflüssigkeit benetzt, so daß zuerst seine dickere Innenschicht 6 aufquillt und den Innenraum verschließt. Mit der gewünschten Verzögerung beginnt auch die Außenschicht 7 des Kollagen-Stöpsels 4 aufzuquellen, wodurch eine Dichtwirkung zwischen dem Rand der Öffnung und der Außenwand des Kollagen-Stöpsels erreicht wird. Zweckmäßig sollte der Daumendruck auf den Applikator 5 etwa 5 min aufrechterhalten werden, um die Lage des Kollagen-Stöpsels 4 während des Quellvorgangs zu sichern.

Im folgenden werden einige Beispiele zur Herstellung der erfindungsgemäßen Vorrichtung erläutert.

### Beispiel 1

Aus Rindleimstoff wird ein Schaum in bekannter Weise erzeugt. In den für die innere Schicht bestimmten Schaumstoff wird kein Härtemittel zugegeben, in den für die äußere Schicht 7 wird vor der Lyophylisation 0,2 % Glutaraldehyd oder Methylglyoxal als Trockengut zugegeben. Nach Bildung des Schaumes wird der für die innere Schicht 6 bestimmte Schaum in angemessener Schichtdicke auf einen starren Kern aus rostfreiem Stahl aufgerollt und auf 20 % des ursprünglichen Volumens gepreßt. Nach der Stabilisation dieses Gebildes wird eine vorbestimmte Schicht des gehärteten Kollagen-Schaums aufgerollt und auf 10 - 20 % des ursprünglichen Volumens gepreßt. Nach der Stabilisation der Außenschicht und gegenseitiger Fixation der beiden Schichten 6, 7 liegt die Wandstärke im trockenen Zustand unter etwa 2 mm. Der Außendurchmesser des starren Kerns entspricht dem Außendurchmesser des Katheters 3 bzw. 16, für den der jeweilige Kollagen-Stöpsel 4 bestimmt ist. Auf dem Kern wird in den beiden Schichten mit einem speziellen Schneidewerkzeug ein Längsschlitz 8 ausgeschnitten und anschließend wird der Kollagen-Stöpsel 4 vom Kern entfernt. Zweckmäßig sollte die Länge des Kollagen-Stöpsels 4 etwa 2 cm betragen, wobei für besondere Anwendungen z. B. bei fettleibigen Patienten auch wesentliche längere Kollagen-Stöpsel 4 Verwendung finden können. Zusammen mit einem starren Applikator 5 von entsprechendem Innendurchmesser werden beide Teile verpackt, entsprechend bezeichnet und durch Bestrahlung sterilisiert.

### Beispiel 2

Aus Rindleimstoff wird ein Schaum in bekannter Weise erzeugt. Vor der Lyophylisation werden in einen Behälter, in welchem die Lyophylisation ausgeführt wird, in die Mitte einer vorzugsweise 10 mm dicken Kollagenschicht Kerne aus rostfreiem Stahl mit einem dem Innendurchmesser des herzustellenden Kollagen-Stöpsels entsprechenden Außendurchmesser plaziert und die Lyophylisation wird durchgeführt. Danach wird der Schaum in Längsrichtung zwischen den Kernen so zerschnitten, daß die den einzelnen Kern umgebende Schaumschicht praktisch gleichmäßig ist. Dieser Schaum wird auf den Kern auf 10 - 20 % seines ursprünglichen Volumens gepreßt. Kerne mit diesem gepreßten Schaum werden vor der Lyophylisation in gleicher Weise in der Mitte einer Schicht aus der härteren Kollagen-Masse angebracht, wobei diese Schicht die Dicke von 6 mm nicht überschreitet. Nach Beendigung der Lyophylisation wird der Schaum wieder länglich zerschnitten, so daß die Schicht des gehärteten Schaumes auf dem gesamten Umfang der ersten Schicht praktisch gleichmäßige Dicke hat. Dann wird diese Schicht auf 10 % ihres ursprünglichen Volumens gepreßt. Nach vollkommener Stabilisation der Form wird der V-förmige Längsschlitz mit einem speziellen Schneidwerkzeug im zweischichtigen resorbierbaren Kollagen-Stöpsel hergestellt und der fertige Kollagen-Stöpsel 4 wird vom Kern abgenommen.

### Beispiel 3

In den Rindleimstoff wird vor der Lyophylisation ein thromboplastisch wirkender Stoff, z. B. menschlicher Thrombin, getrocknet, im Anteil von 100 Einheiten für 1 g Trockengut Kollagen oder 1 % Chitosan, bezogen auf Trockengut, homogen eingemischt.

### Beispiel 4

In den Rindleimstoff wird vor der Lyophylisation ein antibakteriell wirkendes Mittel, z. B. Neomycin, im Anteil von 0,5 g für 1 g Trockengut einhomogenisiert.

### Beispiel 5

Die Herstellung und Zubereitung des Kollagen-Stöpsels ist die gleiche wie in den Beispielen 1 bis 4. Als Ausgangsmaterial wird jedoch statt Rindleimstoff ein aus Rindflechsen erhaltenes Kollagen eingesetzt.

### Beispiel 6

Da der Hauptzweck der äußeren Schicht 7 im Aufrechterhalten der Stabilität des zweischichtigen resorbierbaren Kollagen-Stöpsels 4 während der zum Einführen bis zur Gefäßwand und ggf. zum Aufquellen erforderlichen Zeit besteht, wird eine dickere Innenschicht 6 aus Kollagen-Schaum durch Pressen auf dem Kern gebildet, der etwa 90 % der gesamten Wandstärke besitzt. Die Außenschicht 7 wird dann durch wiederholte Applikation der ausgehärteten Kollagen-Masse oder durch Aufwickeln eines aus dieser Masse gebildeten Films hergestellt. Dann wird das gesamte Gebilde gründlich getrocknet.

Die Erfindung ist nicht auf die dargestellte Vorrichtung beschränkt. So kann der Stöpsel 4 auch aus einem anderen geeigneten Material bestehen, das resorbierbar sein sollte und unter bestimmten Umständen gezielt sein Volumen vergrößern kann. Ferner kann der Stöpsel aus mehr als zwei Schichten bestehen und eine geringfügig kegelige Form haben. Schließlich können an der Außenwand des Stöpsels Mittel vorgesehen sein, die ein zu tiefes Eindringen in das Gefäß und damit die Gefahr eines Verschlusses des gesamten Gefäßes verhindern. Diese Mittel können z. B. als Profilierungen der Außenwand, als Ringwulste z. B. aus dem schnell quellenden weicheren Schaumstoff usw. ausgeführt sein.

## Patentansprüche

1. Vorrichtung zum Verschließen einer durch Einführen eines Katheters verursachten Gefäßöffnung,
- mit einem rohrförmigen Stöpsel (4), dessen Innendurchmesser dem Außendurchmesser des jeweils verwendeten Katheters entspricht, so daß er auf dem Katheter positioniert und bis zur Gefäßöffnung verschoben werden kann,
**dadurch gekennzeichnet,**
- daß der Stöpsel (4) aus mehreren Schichten (7, 8) aus resorbierbarem komprimierten Schaumstoff-Material besteht, das unter Einwirkung von Körperflüssigkeit aufquillt und die Gefäßöffnung verschließt, wobei eine äußere Schaumstoff-Schicht (7) dünnwandiger und formsteifer als eine innere Schaumstoff-Schicht (8) ist, welche schneller als die äußere Schaumstoff-Schicht (7) aufquillt, und
- daß ein hohlzylindrischer Applikator (5; 11 bis 15) aus einem formsteifen biologisch inerten Material vorgesehen ist, dessen Innendurchmesser dem Innendurchmesser des Stöpsels (4) entspricht, so daß er hinter dem Stöpsel (4) auf dem Katheter (3, 16) positioniert werden kann, um den Stöpsel (4) auf dem Katheter bis zur Gefäßöffnung zu verschieben.

2. Vorrichtung nach Anspruch 1, dadurch gekennzeichnet, daß der Stöpsel (4) aus mindestens einem komprimierten Schaumstoff auf der Basis eines fibrillären Eiweißstoffes, wie insbesondere Kollagen, besteht.

3. Vorrichtung nach Anspruch 1 oder 2, dadurch gekennzeichnet, daß der rohrförmige Stöpsel (4) einen vorzugsweise V-förmigen Längsschlitz (8) und an seinem vorderen Ende eine schneidenförmige Abschrägung aufweist.

4. Vorrichtung nach einem der Ansprüche 1 bis 3, dadurch gekennzeichnet, daß der Stöpsel (4) kegelförmig ausgebildet ist, wobei sein außenseitiges Ende A höchstens 20 % breiter als sein inneres Ende ist.

5. Vorrichtung nach einem der Ansprüche 1 bis 4, dadurch gekennzeichnet, daß die Außen- und Innendurchmesser des Stöpsels (4) und des Applikators (5) aufeinander und auf den Außendurchmesser des Katheters (3, 16) abgestimmt sind.

6. Vorrichtung nach einem der Ansprüche 1 bis 5, dadurch gekennzeichnet, daß der rohr- bzw. hülsenförmige Applikator (5) aus Kunststoff oder Edelstahl besteht und einen vorzugsweise V-förmigen durchgehenden Längsschlitz (10) sowie an seinem äußeren Ende einen Ringwulst (9) aufweist.

7. Vorrichtung nach einem der Ansprüche 1 bis 5, dadurch gekennzeichnet, daß der Applikator (11 bis 15) mehrteilig ausgebildet ist und einen auf das Katheter (16) aufschiebbaren Ring (11) zum Verschieben des Stöpsels (4) aufweist, an dem Bauteile (13, 14, 15) zum Aufbringen und Übertragen des für die Verschiebung notwendigen Drucks fixierbar ist.

8. Vorrichtung nach einem der Ansprüche 1 bis 7, dadurch gekennzeichnet, daß der Schaumstoff des Stöpsels (4) eine thrombogen wirkende Substanz, einen RTG-Kontraststoff und/oder ein Antibiotikum enthält.

9. Verfahren zum Herstellen eines Stöpsels für eine Vorrichtung nach einem der Ansprüche 1 bis 8, dadurch gekennzeichnet,
daß auf einem stabförmigen Kern von vorgegebenem Durchmesser eine Schicht aus einem quellfähigen fibrillären Eiweiß-Schaumstoff, z.B. auf Kollagenbasis, in vorbestimmter Wandstärke aufgebracht wird,
daß diese Schicht auf dem Kern zu einer vorbestimmten Wandstärke radial formhaltig komprimiert und lyophilisiert wird,
daß auf dieser komprimierten Schicht eine weitere Schicht aus einem quellfähigen fibrillären Eiweiß-Schaumstoff, z.B. auf Kollagenbasis, in vorbestimmter geringerer Wandstärke aufgebracht wird,
daß diese äußere Schicht zu einer vorbestimmten Wandstärke radial formhaltig komprimiert wird und
daß dieser rohrförmige Körper ggf. nach Herstellung eines V-förmigen Längsschlitzes in die Stöpsel von vorgegebener Länge unterteilt wird.

## Claims

1. Device for closing a vessel opening caused by introduction of a catheter,
- with a tubular plug (4) whose internal diameter corresponds to the external diameter of the particular catheter used, so that it can be positioned on the catheter and advanced as far as the vessel opening,
characterized in that
- the plug (4) is made up of a plurality of layers (7, 8) of absorbable compressed foam material which swells under the influence of body fluid and closes the vessel opening, an outer foam layer (7) having a thinner wall and being more dimensionally stable than an inner foam layer (8), which swells more quickly than the outer foam layer (7), and
- a hollow cylindrical applicator (5; 11 to 15) made of a dimensionally stable, biologically inert material is provided, whose internal diameter corresponds to the internal diameter of the plug (4), so that it can be positioned behind the plug (4) on the catheter (3, 16) in order to advance the plug (4) on the catheter as far as the vessel opening.

2. Device according to Claim 1, characterized in that the plug (4) consists of at least one compressed foam based on a fibrillar protein such as, in particular, collagen.

3. Device according to Claim 1 or 2, characterized in that the tubular plug (4) has a preferably V-shaped longitudinal slot (8) and, at its front end, a bevel in the shape of a cutting edge.

4. Device according to one of Claims 1 to 3, characterized in that the plug (4) is of conical design, its outer end A being at most 20% wider than its inner end.

5. Device according to one of Claims 1 to 4, characterized in that the external and internal diameters of the plug (4) and of the applicator (5) are adapted to one another and to the external diameter of the catheter (3, 16).

6. Device according to one of Claims 1 to 5, characterized in that the tubular or sleeve-shaped applicator (5) is made of plastic or special steel and has a preferably V-shaped continuous longitudinal slot (10) and, at its outer end, an annular bulge(9).

7. Device according to one of Claims 1 to 5, characterized in that the applicator (11 to 15) is of multi-part design and has a ring (11) which can be pushed onto the catheter (16) for advancing the plug (4), and on which components (13, 14, 15) can be fixed for applying and transmitting the pressure necessary for the advancing movement.

8. Device according to one of Claims 1 to 7, characterized in that the foam of the plug (4) contains a substance with thrombogenic action, an X-ray contrast agent and/or an antibiotic.

9. Method for producing a plug for a device according to one of Claims 1 to 8, characterized in that
a layer of a swellable fibrillar protein foam, e.g. based on collagen, is applied in a predetermined wall thickness to a rod-shaped core of predetermined diameter,
this layer, while retaining its shape, is radially compressed on the core until it reaches a predetermined wall thickness, and is lyophilized,
a further layer of a swellable fibrillar protein foam, e.g. based on collagen, is applied in a predetermined smaller wall thickness to this compressed layer,
this outer layer, while retaining its shape, is radially compressed until it reaches a predetermined wall thickness, and
this tubular body, if appropriate after a V-shaped longitudinal slot has been made, is subdivided into the plugs of predetermined length.

## Revendications

1. Dispositif pour obstruer une ouverture dans un vaisseau, provoquée par l'introduction d'un cathéter,
- avec un bouchon (4) de forme tubulaire, dont le diamètre intérieur correspond au diamètre extérieur du cathéter chaque fois utilisé, si bien qu'il peut être positionné sur le cathéter et être coulissé jusqu'à l'ouverture du vaisseau,
caractérisé en ce que,
- le bouchon (4) est constitue de plusieurs couches (7, 8) en un matériau alvéolaire comprimé résorbable, qui gonfle sous l'effet du liquide corporel et obstrue l'ouverture de vaisseau, une couche extérieure de matériau alvéolaire (7) étant d'épaisseur de paroi plus faible et de rigidité de forme plus grande qu'une couche intérieure de matériau alvéolaire (8) qui gonfle plus rapidement que la couche de matériau alvéolaire (7), et
- en ce qu'est prévu un applicateur cylindrique creux (5; 11 à 15), constitué d'un matériau inerte biologiquement et à rigidité de forme, dont le diamètre intérieur correspond au diamètre intérieur du bouchon (4), si bien qu'il peut être positionné derrière le bouchon (4) sur le cathéter (3, 16) pour faire coulisser le bouchon (4) sur le cathéter jusqu'à l'ouverture du vaisseau.

2. Dispositif selon la revendication 1, caractérisé en ce que le bouchon (4) est constitué d'au moins un matériau alvéolaire comprimé à base d'albumine fibrillaire, tel qu'en particulier du collagène.

3. Dispositif selon la revendication 1 ou 2, caractérisé en ce que le bouchon à forme tubulaire (4) présente une fente longitudinale (8) de préférence en forme de "V" et présente à son extrémité un chanfreinage en forme de tranchant.

4. Dispositif selon l'une des revendications 1 à 3, caractérisé en ce que le bouchon (4) est réalisé à forme conique, son extrémité A située du côté extérieur étant au plus de 20 % plus large que son extrémité intérieure.

5. Dispositif selon l'une des revendications 1 à 4, caractérisé en ce que le diamètre extérieur et intérieur du bouchon (4) et de l'applicateur (5) sont conçus l'un en fonction de l'autre et en fonction du diamètre extérieur du cathéter (3, 16).

6. Dispositif selon l'une des revendications 1 à 5, caractérisé en ce que l'applicateur (5) en forme de tube, respectivement de douille est réalisé en matière plastique ou en acier spécial et présente une fente longitudinale (10) continue, de préférence en forme de "V", ainsi que sur son extrémité extérieure un bourrelet annulaire (9).

7. Dispositif selon l'une des revendications 1 à 5, caractérisé en ce que l'applicateur (11 à 15) est réalisé en plusieurs parties et présente un anneau (11) pouvant coulisser sur le cathéter (16), pour déplacer le bouchon (4), auquel des composants (13, 14, 15) destinés à l'application et à la transmission de la pression nécessaire pour le coulissement peuvent être fixés.

8. Dispositif selon l'une des revendications 1 à 7, caractérisé en ce que le matériau alvéolaire du bouchon (4) contient une substance à effet thrombogène, une substance à constraste aux rayons X et/ou un antibiotique.

9. Procédé de réalisation d'un bouchon pour un dispositif selon l'une des revendications 1 à 8, caractérisé
en ce que, sur un noyau en forme de barre ayant un diamètre prédéterminé, on applique une couche d'un matériau alvéolaire à base d'albumine fibrillaire, susceptible de gonfler, par exemple à base de collagène, en une épaisseur de paroi prédéterminée,
en ce que cette couche est comprimée en conservant sa forme, radialement, sur le noyau, jusqu'à obtention d'une épaisseur de paroi prédéterminée et est lyophilisée,
en ce que sur cette couche comprimée est appliquée une autre couche constituée d'un matériau à base d'albumine fibrillaire, susceptible de gonfler, par exemple à base de collagène, en une épaisseur de paroi inférieure prédéterminée,
en ce que la couche extérieure est comprimée, en conservant la forme, radialement, jusqu'à obtention d'une épaisseur de paroi prédéterminée, et
en ce que ce corps à forme tubulaire est subdivisé, le cas échéant après réalisation d'une fente longitudinale en forme de "V", en formant les bouchons de longueur prédéterminée.
